# EUROPEAN PATENT APPLICATION

(11) **EP 4 360 593 A2**
(43) Date of publication of application: **01.05.2024**
(21) Application number: 23199179.5
(22) Date of filing: 22.09.2023
(51) Int. Cl.: A61F 2/24

(54) **PROSTHETIC HEART VALVE WITH NON-ABRASIVE ATTACHMENTS**

(30) Priority: 03.10.2022 US 202263412781 P; 06.09.2023 US 202318462348
(71) Applicant: Medtronic Vascular, Inc., Santa Rosa, CA 95403 (US)
(72) Inventor: Dinh, Thuy Linh L., Santa Ana, 92705 (US); Kibria, Alkindi, Santa Ana, 92705 (US); Cheshko, Tasha, Santa Ana, 92705 (US); Dorman, Kyle J., Santa Ana, 92705 (US); Goshgarian, Justin G., Santa Rosa, 95403 (US)
(74) Representative: Zimmermann & Partner Patentanwälte mbB

(57) **Abstract**

The techniques of this disclosure generally relate to a valve prosthesis include a prosthetic valve, a stent structure having suture slots, an outer skirt, and outer skirt sutures. The outer skirt sutures attach the outer skirt to the stent structure. The outer skirt sutures are located within the suture slots. By recessing the outer skirt sutures within the suture slots below an inner surface of the stent structure, contact between the outer skirt sutures and valve leaflets of the prosthetic valve is avoided. By avoiding contact between the outer skirt sutures and the valve leaflets, damage to the valve leaflets due to abrasive contact with the outer skirt sutures is prevented.

## Description

### CROSS-REFERENCE TO RELATIVE APPLICATIONS

This application claims the benefit of U.S. Provisional Application No. 63/412,781, filed October 3, 2022, the contents of which are incorporated by reference herein in their entirety.

### FIELD

The present technology is generally related to transcatheter heart valves.

### BACKGROUND

A human heart includes four heart valves that determine the pathway of blood flow through the heart: the mitral valve, the tricuspid valve, the aortic valve, and the pulmonary valve. The mitral and tricuspid valves are atrioventricular valves, which are between the atria and the ventricles, while the aortic and pulmonary valves are semilunar valves, which are in the arteries leaving the heart. Ideally, native leaflets of a heart valve move apart from each other when the valve is in an open position, and meet or "coapt" when the valve is in a closed position. Problems that may develop with valves include stenosis in which a valve does not open properly, and/or insufficiency or regurgitation in which a valve does not close properly. Stenosis and insufficiency may occur concomitantly in the same valve. The effects of valvular dysfunction vary, with regurgitation or backflow typically having relatively severe physiological consequences to the patient.

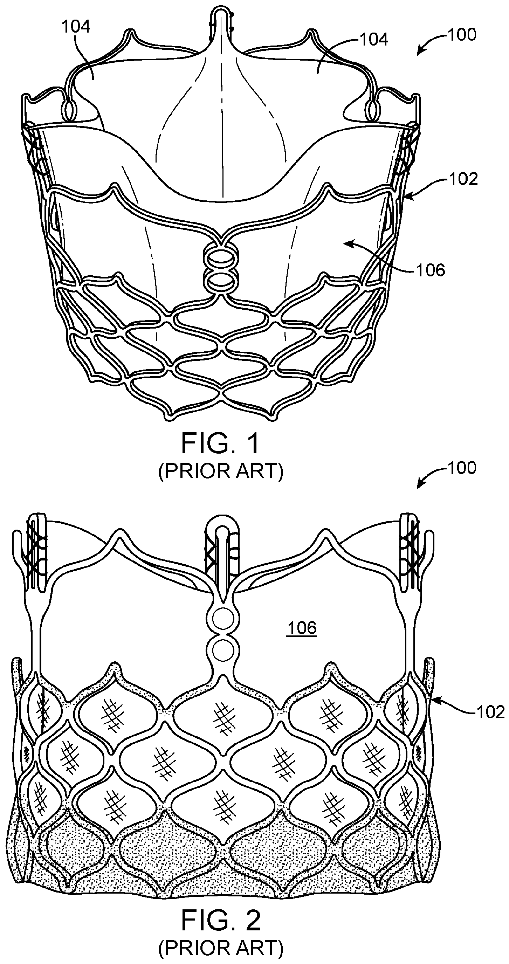

Recently, flexible prosthetic valves supported by stent structures that can be delivered percutaneously using a catheter-based delivery system have been developed for heart and venous valve replacement. FIG. 1 is a perspective view of a valve prosthesis 100 in accordance with the prior art. FIG. 2 is a side view of valve prosthesis 100 of FIG. 1 in accordance with the prior art. Referring to FIGS. 1 and 2, valve prosthesis 100 may include a balloon-expandable stent structure 102 with valve leaflets 104 attached to the interior of stent structure 102. Valve leaflets 104 may be parts of a 3D single piece prosthetic valve 106.

Valve prosthesis 100 can be reduced in diameter, by crimping onto a balloon catheter, and advanced through the venous or arterial vasculature. Once valve prosthesis 100 is positioned at the treatment site, for instance within an incompetent native valve, stent structure 102 may be expanded to hold valve prosthesis 100 firmly in place.

When designing a valve prosthesis such as valve prosthesis 100, valve-frame integration and frame mechanical performance often have competing needs or requirements. For example, when attaching the valve to the frame during valve-frame integration, the valve itself needs to be reinforced to the frame at certain locations without hindering mechanical performance of the frame. Embodiments hereof relate to an improved balloon-expandable transcatheter valve prosthesis configured to minimize tradeoffs between the above-described competing needs.

### SUMMARY

The techniques of this disclosure generally relate to a valve prosthesis include a prosthetic valve, a stent structure having suture slots, an outer skirt, and outer skirt sutures. The outer skirt sutures attach the outer skirt to the stent structure. The outer skirt sutures are located within the suture slots. By recessing the outer skirt sutures within the suture slots below an inner surface of the stent structure, contact between the outer skirt sutures and valve leaflets of the prosthetic valve is avoided. By avoiding contact between the outer skirt sutures and the valve leaflets, damage to the valve leaflets due to abrasive contact with the outer skirt sutures is prevented.

In one aspect, the present disclosure provides a valve prosthesis include a prosthetic valve, a stent structure having suture holes, an outer skirt, and outer skirt sutures. The outer skirt sutures attach the outer skirt to the stent structure. The outer skirt sutures are located within the suture holes. By placing the outer skirt sutures within the suture holes and away from an inner surface of the stent structure, contact between the outer skirt sutures and valve leaflets of the prosthetic valve is avoided. By avoiding contact between the outer skirt sutures and the valve leaflets, damage to the valve leaflets due to abrasive contact with the outer skirt sutures is prevented.

In another aspect, the present disclosure provides a valve prosthesis include a prosthetic valve having valve leaflets and a valve inflow cylinder proximal of the valve leaflets. The valve prosthesis further includes a stent structure having suture apertures extending radially through the stent structure and tacking stitches. The tacking stitches tack the prosthetic valve to the stent structure and are located within the suture apertures. By tacking the prosthetic valve to the stent structure with the tacking stitching through the suture openings, the prosthetic valve is attached to stent structure securely while at the same time minimizing stress of the connection.

In another aspect, the present disclosure provides a valve prosthesis having a prosthetic valve, a stent structure, and an outer skirt. The stent structure includes an inflow portion having inflow portion cells. The outer skirt is collagen bonded to the prosthetic valve through the inflow portion cells to mount the prosthetic valve to the stent structure. By using collagen bonding to mount the prosthetic valve to the stent structure instead of stitches, damage to valve leaflets of the prosthetic valve from stitches is avoided.

In another aspect, the present disclosure provides a method including collagen bonding a prosthetic valve to an outer skirt through a stent structure by pressing the prosthetic valve against the outer skirt with the stent structure in between the prosthetic valve and the outer skirt.

Further disclosed herein is a valve prosthesis including a prosthetic valve, a stent structure having suture slots, an outer skirt, and outer skirt sutures. The outer skirt sutures attach the outer skirt to the stent structure. The outer skirt sutures are located within the suture slots. By recessing the outer skirt sutures within the suture slots below an inner surface of the stent structure, contact between the outer skirt sutures and valve leaflets of the prosthetic valve is avoided. By avoiding contact between the outer skirt sutures and the valve leaflets, damage to the valve leaflets due to abrasive contact with the outer skirt sutures is prevented.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1 is a perspective view of a valve prosthesis in accordance with the prior art.
FIG. 2 is a side view of the valve prosthesis of FIG. 1 in accordance with the prior art.
FIG. 3 is a side view of a valve prosthesis in an expanded configuration in accordance with one embodiment.
FIG. 4 is an outflow end view of a prosthetic valve of the valve prosthesis of FIG. 3 in accordance with one embodiment.
FIG. 5 is a perspective view of the prosthetic valve of the valve prosthesis of FIG. 3 in accordance with one embodiment.
FIG. 6 is a cross-sectional view of the valve prosthesis along the line VI-VI of FIG. 3 in accordance with one embodiment.
FIG. 7 is a plan view of a stent structure of the valve prosthesis from the direction of arrow VII of FIG. 6 in accordance with one embodiment.
FIG. 8 is an enlarged cross-sectional view of a region VIII of the valve prosthesis of FIG. 6 in accordance with one embodiment.
FIG. 9 is a cross-sectional view of the valve prosthesis along the line VI-VI of FIG. 3 in accordance with another embodiment.
FIG. 10 is a side view of a valve prosthesis in an expanded configuration in accordance with one embodiment.
FIG. 11 is a perspective view of a prosthetic valve including tacking stitching of the valve prosthesis of FIG. 10 in accordance with one embodiment.
FIG. 12 is an enlarged plan view of a region XII of the valve prosthesis of FIG. 10 in accordance with one embodiment.
FIG. 13 is an enlarged plan view of a region XIII of the valve prosthesis of FIG. 10 in accordance with one embodiment.
FIG. 14 is a side view of a valve prosthesis in an expanded configuration in accordance with one embodiment.
FIG. 15 is cross-sectional view of along the line XV-XV of the valve prosthesis of FIG. 14 in accordance with one embodiment.

### DETAILED DESCRIPTION

Specific embodiments are now described with reference to the figures, wherein like reference numbers indicate identical or functionally similar elements. The terms "distal" and "proximal", when used in the following description to refer to a native vessel, native valve, or a device to be implanted into a native vessel or native valve, such as a heart valve prosthesis, are with reference to the direction of blood flow. Thus, "distal" and "distally", also referred to as the "outflow" or "outflow direction", respectively, refer to positions in a downstream direction with respect to the direction of blood flow. The terms "proximal" and "proximally", also referred to as the "inflow" or "inflow direction", respectively, refer to positions in an upstream direction with respect to the direction of blood flow. Proximal is also sometimes referred to as inferior and distal is sometimes referred to as superior.

Although the description is in the context of treatment of an aortic heart valve, embodiments may also be used where it is deemed useful in other valved intraluminal sites that are not in the heart. For example, embodiments may be applied to other heart valves or venous valves as well.

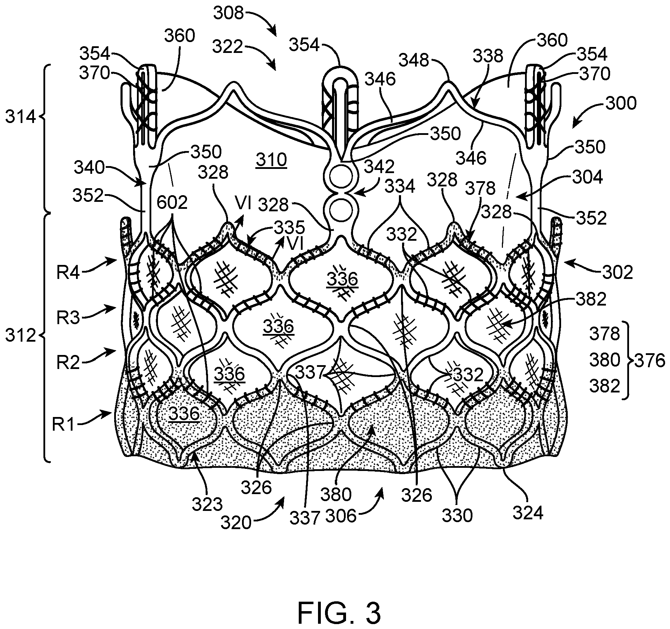

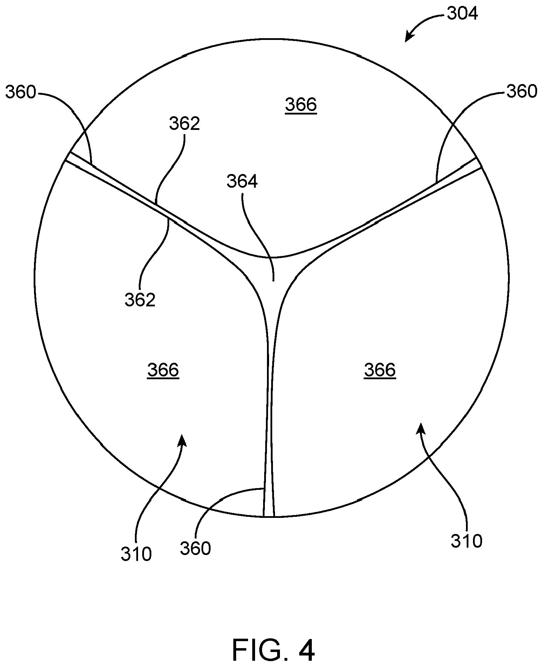

FIG. 3 is a side view of a transcatheter valve prosthesis 300 in the expanded configuration in accordance with one embodiment. FIG. 4 is an outflow end view of a prosthetic valve 304 of valve prosthesis 300 of FIG. 3 in accordance with one embodiment. Valve prosthesis 300 has a radially-expandable stent structure 302, sometimes called the frame, and prosthetic valve 304. Stent structure 302 is generally tubular, and is mechanically or balloon expandable, having a crimped configuration for delivery within a vasculature and an expanded configuration for deployment within a native heart valve. When valve prosthesis 300 is deployed within the valve annulus of a native heart valve, stent structure 302 of valve prosthesis 300 is configured to be radially expanded within native valve leaflets of the patient's defective valve, to thereby retain the native valve leaflets in a permanently open state. In embodiments hereof, valve prosthesis 300 is configured for replacement of an aortic valve such that an inflow end 306, i.e., the proximal end, of valve prosthesis 300 extends into and anchors within the aortic annulus of a patient's left ventricle, while an outflow end 308, i.e., the distal end, of valve prosthesis 300 is positioned within the aortic sinuses.

Stent structure 302 of valve prosthesis 300 may be a unitary frame or scaffold that supports prosthetic valve 304 including one or more valve leaflets 310 within the interior of stent structure 302. Prosthetic valve 304 is capable of blocking flow in one direction to regulate flow there-through via valve leaflets 310 that may form a bicuspid or tricuspid replacement valve. FIG. 4 illustrates that prosthetic valve 304 has three valve leaflets 310, i.e., prosthetic valve 304 has a tricuspid leaflet configuration, although a bicuspid leaflet configuration may be used in other embodiments. More particularly, as valve prosthesis 300 is configured for placement within a native aortic valve which typically has three leaflets, prosthetic valve 304 may include three valve leaflets 310. However, valve prosthesis 300 is not required to have the same number of leaflets as the native valve. If valve prosthesis 300 is alternatively configured for placement within a native valve having two leaflets such as the mitral valve, prosthetic valve 304 may include two or three valve leaflets 310.

Stent structure 302 is balloon-expandable. As such, stent structure 302 is made from a plastically deformable material such that when expanded by a dilatation balloon, stent structure 302 maintains its radially expanded configuration. Stent structure 302 may be formed from stainless steel such as 316L or other suitable metal, such as platinum iridium, cobalt chromium alloys such as MP35N or L605, or various types of polymers or other similar materials, including the materials coated with various surface deposits to improve clinical functionality. Stent structure 302 is configured to be rigid such that it does not deflect or move when subjected to in-vivo forces, or such that deflection or movement is minimized when subjected to in-vivo forces.

Stent structure 302 includes an inflow portion 312 and an outflow portion 314. Stent structure 302 is a tubular component defining a central lumen or passageway, and has an inflow end 320 and an outflow end 322. When expanded, a diameter of inflow end 320 of stent structure 302 is substantially the same as a diameter of outflow end 322 of stent structure 302 in one embodiment.

Inflow portion 312 extends distally from inflow end 320 of stent structure 302. Inflow portion 312 includes inflow crowns 324, central crowns 326, outflow crowns 328, inflow struts 330, central struts 332, and outflow struts 334. Inflow portion 312 further includes an inflow crown ring 323 defined by inflow crowns 324, inflow struts 330, and central crowns 326. Inflow portion further includes an outflow crown ring 335 defined by outflow crowns 328, outflow struts 334, and central crowns 326. Inflow portion 312 generally extends between inflow crown ring 323 and outflow crown ring 335.

In between inflow crown ring 323 and outflow crown ring 335, inflow portion cells 336, sometimes called side openings, are formed in rows, and more particularly, in four rows R1, R2, R3, and R4. Each row R1, R2, R3, R4 includes 12 inflow portion cells 336. Inflow portion cells 336 of the proximal most row R1 are defined by inflow crowns 324, inflow struts 330, central crowns 326, and central struts 332. Inflow portion cells 336 of the next most proximal rows R2, R3 are defined by central crowns 326 and central struts 332. Inflow portion cells 336 of the distal most row R4 are defined by outflow crowns 328, outflow struts 334, central crowns 326, and central struts 332. Generally, inflow portion cells 336 are diamond-shaped openings having the same or identical shaped, sometimes are called symmetric.

Generally, a crown is defined where two struts connect and a node is defined as a region where two crowns connect. Accordingly, inflow crowns 324 are defined where inflow struts 330 connect. Outflow crowns 328 are defined where outflow struts 334 connect. Central crowns 326 are defined where inflow struts 330 connect, where outflow struts 334 connect, and where central struts 332 connect. Central nodes 337 are defined where central crowns 326 connect.

Outflow portion 314 is formed proximate to outflow end 322 of stent structure 302 and between outflow end 322 and inflow portion 312. Outflow portion 314 includes an outflow crown ring 338, commissure posts 340, and non-commissure posts 342. Outflow portion 314 can be configured in a shape that forms a central lumen or passageway.

Outflow crown ring 338 includes outflow crown struts 346, superior crowns 348, and inferior crowns 350. Each outflow crown struts 346 is connected on one end to an adjacent outflow crown strut 346 at a superior crown 348 and on the opposite end to an adjacent outflow crown strut 346 at an inferior crown 350. Inferior crowns 350 are connected to either a commissure post 340 or a non-commissure post 342. More particularly, every other inferior crown 350 is connected to a commissure post 340 and every other inferior crown 350 is connected to a non-commissure post 342 in an alternating repeating arrangement.

Non-commissure posts 342, sometimes called axial frame members 342, extend longitudinally between and connect inferior crowns 350 of outflow crown ring 338 and outflow crowns 328 of inflow portion 312. In accordance with this embodiment, non-commissure posts 342 are shaped as a figure eight and can be used as markers for depth of implant as well as clocking of valve prosthesis 300. As used herein, longitudinally is in a direction parallel with the longitudinal axis, radially is perpendicular and in a radial direction from the longitudinal axis, and circumferentially is in a plane perpendicular to the longitudinal axis and in a direction along the circumference of valve prosthesis 300.

Commissure posts 340 extend longitudinally and include axial frame members 352 and trident posts 354. Axial frame members 352 extend longitudinally between and connect inferior crowns 350 of outflow crown ring 338 and outflow crowns 328 of inflow portion 312. Trident posts 354 extend in a cantilever fashion and in the outflow or distal direction from inferior crowns 350 of outflow crown ring 338. Axial frame members 352 and trident posts 354 are parallel with one another and are segments of commissure posts 340, which are linear members.

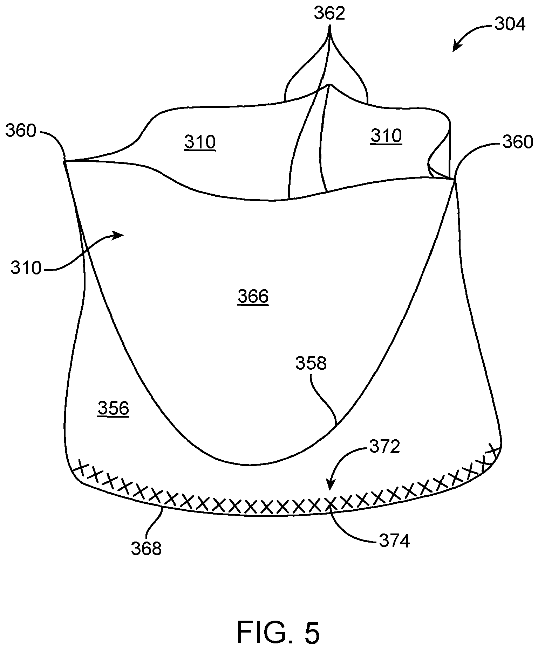

FIG. 5 is a perspective view of prosthetic valve 304 of valve prosthesis 300 of FIG. 3 in accordance with one embodiment. FIGS. 3, 5 illustrate prosthetic valve 304 in an open state whereas FIG. 4 illustrates prosthetic valve 304 in a closed state.

Referring now to FIGS. 3-5 together, prosthetic valve 304 is a one piece three dimensional (3D) molded structure in accordance with this embodiment. Illustratively, a single cylindrical piece is molded to form prosthetic valve 304. Accordingly, although various structures of prosthetic valve 304 are discussed below, prosthetic value 304 is integral, i.e., formed from a single piece and not a plurality of separate pieces connected together.

Prosthetic valve 304 may be made of pericardial material; however, may instead be made of another material. Natural tissue for prosthetic valve 304 may be obtained from, for example, heart valves, aortic roots, aortic walls, aortic leaflets, pericardial tissue, such as pericardial patches, bypass grafts, blood vessels, intestinal submucosal tissue, umbilical tissue and the like from humans or animals. Synthetic materials suitable for use as prosthetic valve 304 include DACRON polyester commercially, other cloth materials, nylon blends, polymeric materials, and vacuum deposition nitinol fabricated materials. One polymeric material from which prosthetic valve 304 can be made is an ultra-high molecular weight polyethylene material. With certain materials, it may be desirable to coat one or both sides of prosthetic valve 304 with a material that will prevent or minimize overgrowth. It is further desirable that the material is durable and not subject to stretching, deforming, or fatigue.

Prosthetic valve 304 includes valve leaflets 310 and a valve inflow cylinder 356 proximal of valve leaflets 310. For example, valve inflow cylinder 356 is the remaining un-molded portion of the cylindrical material used to form prosthetic valve 304 and valve leaflets 310 are the molded portion.

Valve leaflets 310 are defined by cusps 358, commissures 360, and free edges 362. Adjoining pairs of valve leaflets 310 are attached to one another at their lateral ends to form commissures 360, with free edges 362 of valve leaflets 310 forming coaptation edges that meet in an area of coaptation 364. The region within cusps 358, commissures 360, and free edges 362 are sometimes referred to as a belly 366 of valve leaflets 310.

Valve inflow cylinder 356 has a cylindrical inflow end 368, sometimes called a nadir 368. Valve inflow cylinder 356 extends in the outflow direction from inflow end 368 as a cylinder to cusps 358, which form the outflow end of valve inflow cylinder 356.

Prosthetic valve 304 is disposed within and secured to at least trident posts 354 of commissure posts 340 of stent structure 302. In addition, prosthetic valve 304 may also be disposed within and secured to inflow portion 312 of stent structure 302. More particularly, commissures 360 are attached to trident posts 354, e.g., with commissure stitching 370. Further, a margin of attachment (MOA) 372 of valve inflow cylinder 356, e.g., a region directly adjacent inflow end 368, is attached to inflow portion 312, e.g., with MOA stitching 374. Although a particular location for MOA 372 adjacent inflow end 368 is illustrated in FIG. 5 and discussed above, in other embodiments, MOA 372 is in a different location. For example, MOA 372 is a parabolic MOA adjacent cusps 358. Other locations for MOA 372 are possible in yet other embodiments.

Valve prosthesis 300 further includes a skirt 376, e.g., formed of graft material, which encloses or lines a portion of stent structure 302. Margin of attachment 372 of valve inflow cylinder 356 is sutured or otherwise securely and sealingly attached to the interior surface of skirt 376 and to inflow portion 312, e.g., with MOA stitching 374.

Skirt 376 may enclose or line stent structure 302. Skirt 376 may be a natural or biological material such as pericardium or another membranous tissue such as intestinal submucosa. Alternatively, skirt 376 may be a low-porosity woven fabric, such as polyester, Dacron fabric, or PTFE, which creates a one-way fluid passage. In one embodiment, skirt 376 may be a knit or woven polyester, such as a polyester or PTFE knit, which can be utilized when it is desired to provide a medium for tissue ingrowth and the ability for the fabric to stretch to conform to a curved surface. Polyester velour fabrics may alternatively be used, such as when it is desired to provide a medium for tissue ingrowth on one side and a smooth surface on the other side.

In accordance with this embodiment, skirt 376 includes an outflow end outer skirt 378, and inflow end outer skirt 380, and an inner skirt 382. Outflow end outer skirt 378 is located on the outer surface of outflow crowns 328 and outflow struts 334 of inflow portion 312. Inflow end outer skirt 380 is located on the outer surface of inflow crowns 324, inflow struts 330, central crowns 326, and central struts 332. More particularly, inflow end outer skirt 380 covers first row R1 of inflow portion cells 336. Inner skirt 382 is located on the inner surface of inflow portion 312 and extends between inflow crown ring 323 and outflow crown ring 335.

Delivery of valve prosthesis 300 may be accomplished via a percutaneous transfemoral approach or a transapical approach directly through the apex of the heart via a thoracotomy, or may be positioned within the desired area of the heart via different delivery methods known in the art for accessing heart valves. During delivery, valve prosthesis 300 remains compressed until it reaches a target diseased native heart valve, at which time a balloon of a delivery system is inflated in order to radially expand valve prosthesis 300 in situ. Valve prosthesis 300 is configured to be expanded within the native valve leaflets of the patient's defective valve, to thereby retain the native valve leaflets in a permanently open state. The delivery system is then removed and transcatheter valve prosthesis 300 remains deployed within the native target heart valve.

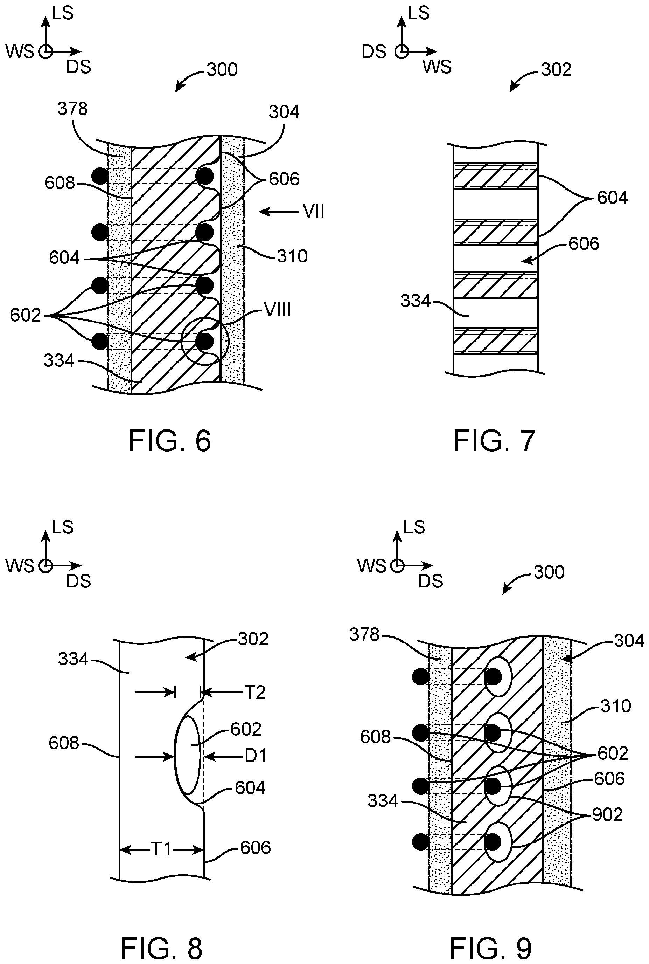

FIG. 6 is a cross-sectional view of valve prosthesis 300 along the line VI-VI of FIG. 3 in accordance with one embodiment. FIG. 7 is a plan view of stent structure 302 of valve prosthesis 300 from the direction of arrow VII of FIG. 6 in accordance with one embodiment. FIG. 8 is an enlarged cross-sectional view of a region VIII of valve prosthesis 300 of FIG. 6 in accordance with one embodiment. Referring now to FIGS. 3-8 together, outflow end outer skirt 378 is attached to stent structure 302 by outer skirt sutures 602.

To accommodate outer skirt sutures 602, stent structure 302 includes suture slots 604 on an inner surface 606 of stent structure 302. Suture slots 604 extend inward into stent structure 302 from inner surface 606 towards an outer surface 608 of stent structure 302 but not entirely through stent structure 302. Suture slots 604 are sometimes called channels, slots, ridges, or indentations. Inner surface 606 and an outer surface 608 are sometimes called the inner diameter (ID) and outer diameter (OD) surfaces, respectively, of stent structure 302.

In accordance with this embodiment, suture slots 604 are formed in outflow struts 334 of inflow portion 312 of stent structure 302. Outer skirt sutures 602 are located within suture slots 604, extend radially outward and around outflow struts 334 and pass radially in and out of outflow end outer skirt 378. Accordingly, outer skirt sutures 602 attach outflow end outer skirt 378 to outflow struts 334, i.e., to outer surface 608 of outflow struts 334.

Paying particular attention to FIGS. 6 and 7, outflow struts 334 have a length direction LS of the length of outflow struts 334, a width direction WS of the width of outflow struts 334, and a depth direction DS of the depth of outflow struts 334. Length direction LS is the direction in which outflow struts 334 extend between respective outflow crowns 328 and central crowns 326 (see FIGS. 3, 6). Length direction LS is perpendicular to both width direction WS and depth direction DS. Width direction WS is perpendicular to depth direction DS. Depth direction DS is in the radial direction of valve prosthesis 300. In one embodiment, directions LS, WS, and DS correspond to the X, Y, and Z axis of a cartesian coordinate system.

In accordance with this embodiment, suture slots 604 extend in the width direction WS across the width of outflow struts 334. Suture slots 604 extend a depth D1 in depth direction DS into outflow struts 334 less than thickness T1 of outflow struts 334 in the depth direction DS. Although suture slots 604 extend perpendicularly across outflow strut 334 in the width direction WS in this embodiment, in other embodiments, suture slots 604 are angled across outflow strut 334 such than an angle exists between suture slots 604 and the width direction WS.

Outer skirt sutures 602 are located in suture slots 604 to be recessed below inner surface 606 of outflow struts 334. Accordingly, outer skirt sutures 602 are radially outward of inner surface 606 of outflow struts 334 and recessed within outflow struts 334.

More particularly, depth D1 of suture slots 604 is greater than a thickness T2 of outer skirt sutures 602 in depth direction DS. Illustratively, depth D1 is 0.15 mm and thickness T2 is less than or equal to 0.15 mm. For example, the thickness (diameter) of outer skirt sutures 602 is in the range of 0.10-0.16 mm in a relaxed and non-compressed cylindrical state. When tightened around outflow struts 334, as illustrated in FIG. 8, outer skirt sutures 602 are deformed to have an elliptical cross-sectional shape having a minor axis in the depth direction DS and a major axis in the length direction LS of outflow struts 334. The minor axis has thickness T2 less than or equal to depth D 1 of suture slots 604.

By recessing outer skirt sutures 602 within suture slots 604, contact between outer skirt sutures 602 and valve leaflets 310 is avoided. By avoiding contact between outer skirt sutures 602 and valve leaflets 310, damage to valve leaflets 310 due to abrasive contact with outer skirt sutures 602 is prevented.

FIG. 9 is a cross-sectional view of valve prosthesis 300 along the line VI-VI of FIG. 3 in accordance with another embodiment. The embodiment of FIG. 9 is similar to the embodiment of FIGS. 6-8 except that suture holes 902 are formed within outflow struts 334 in FIG. 9 instead of sutures slots 604 of FIGS. 6-8.

More particularly, suture holes 902 are formed within outflow struts 334 and extend through outflow struts 334 in the width direction WS. Stated another way, suture holes 902 are radially between inner surface 606 and outer surface 608 in depth direction DS.

Generally, suture holes 902 are larger than outer skirt sutures 602 allowing outer skirt sutures 602 to pass through suture holes 902. In one particular embodiment, suture holes 902 have a diameter of 0.30 mm and the needle used to insert outer skirt sutures 602 into suture holes 902 is 0.30 mm.

Outer skirt sutures 602 are located in suture holes 902 to be below inner surface 606 of outflow struts 334. Accordingly, outer skirt sutures 602 are radially outward of inner surface 606 of outflow struts 334 and within outflow struts 334.

By placing outer skirt sutures 602 within outflow struts 334 and away from inner surface 606, contact between outer skirt sutures 602 and valve leaflets 310 is avoided. By avoiding contact between outer skirt sutures 602 and valve leaflets 310, damage to valve leaflets 310 due to abrasive contact with outer skirt sutures 602 is prevented.

Although FIGS. 6-9 illustrate outer skirt sutures 602 and suture slots 604/suture holes 902 on outflow struts 334, referring now to FIG. 3, outer skirt sutures 602 and suture slots 604/suture holes 902 are placed at other various locations on inflow portion 314 in other embodiments. In one embodiment, outer skirt sutures 602 and suture slots 604/suture holes 902 are placed on outflow crown ring 335 distal of fourth row R4 of inflow portion cells 336 and/or at central struts 332 distal of first row R1 of inflow portion cells 336 as illustrated in FIG. 3. In another embodiment, outer skirt sutures 602 and suture slots 604/suture holes 902 are placed at central struts 332 proximal of fourth row R4 of inflow portion cells 336, e.g., in the case where outflow end outer skirt 378 covers fourth row R4 of inflow portion cells 336.

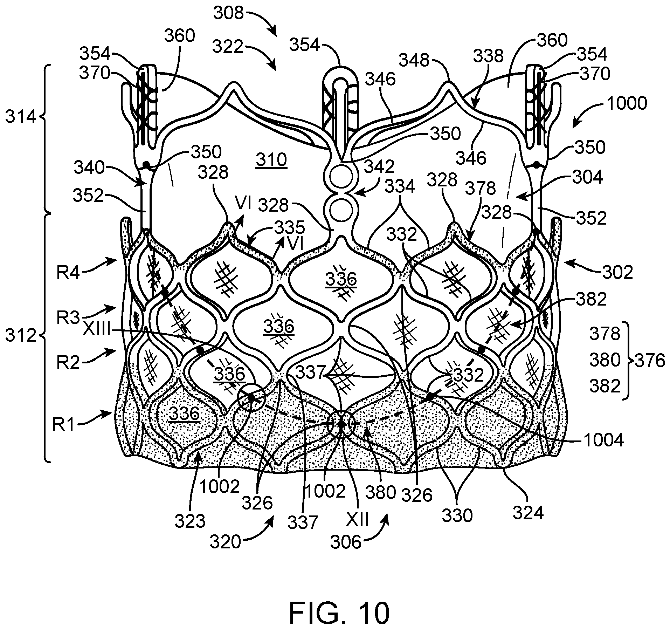

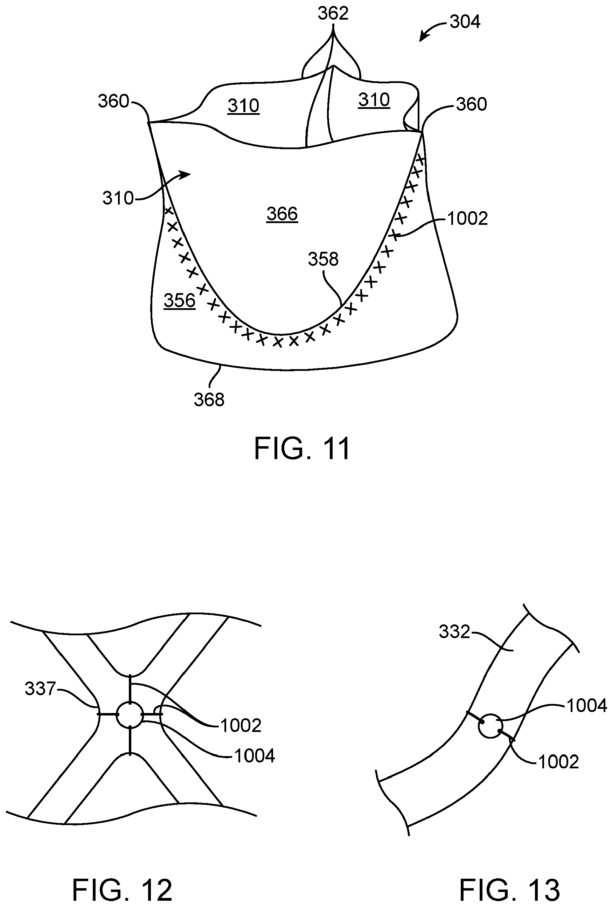

FIG. 10 is a side view of a valve prosthesis 1000 in an expanded configuration in accordance with one embodiment. FIG. 11 is a perspective view of a prosthetic valve 304 including tacking stitching 1002 of valve prosthesis 1000 of FIG. 10 in accordance with one embodiment. FIG. 12 is an enlarged plan view of a region XII of valve prosthesis 1000 of FIG. 10 in accordance with one embodiment. FIG. 13 is an enlarged plan view of a region XIII of valve prosthesis 1000 of FIG. 10 in accordance with one embodiment. Valve prosthesis 1000 of FIG. 10 is similar to valve prosthesis 300 of FIG. 3 and only the significant differences are discussed below.

Referring now to FIGS. 10-13 together, in accordance with this embodiment, suture apertures 1004, sometimes called slots or openings, are formed in stent structure 302. Suture apertures 1004 extend radially and entirely through stent structure 302. In one embodiment, suture apertures 1004 are circular and have a diameter in the range of 0.3 to 1.0 mm.

Suture apertures 1004 are formed in central nodes 337 as illustrated in FIG. 12, in central struts 332 as illustrated in FIG. 13, and/or at other locations on stent structure 302 as needed for any particular stitching pattern of tacking stitching 1002.

Tacking stitching 1002 is passed through suture apertures 1004 to tack prosthetic valve 304 to stent structure 302. Tacking stitching 1002 provide additional tacking to avoid undesirables bulging of prosthetic valve 304, e.g., bulging inward and away from stent structure 302. In accordance with this embodiment, tacking stitching 1002 is provided on valve inflow cylinder 356 adjacent and proximal of cusps 358 as shown in FIG. 11.

By tacking prosthetic valve 304 to stent structure 302 with tacking stitching 1002 through suture apertures 1004, prosthetic valve 304 is attached to stent structure 302 securely while at the same time minimizing stress of the connection.

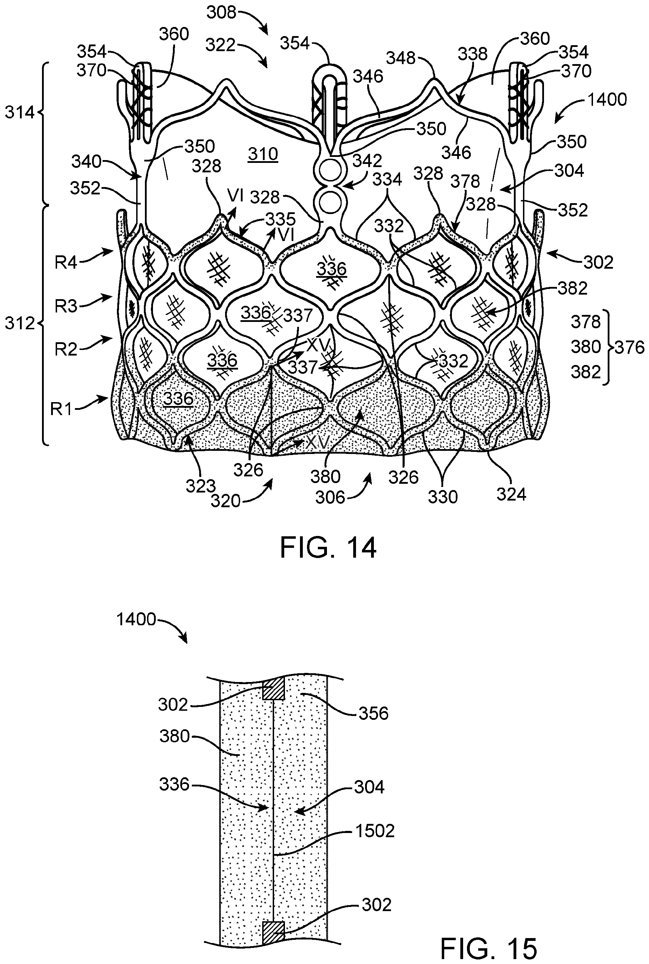

FIG. 14 is a side view of a valve prosthesis 1400 in an expanded configuration in accordance with one embodiment. FIG. 15 is cross-sectional view of along the line XV-XV of valve prosthesis 1400 of FIG. 14 in accordance with one embodiment. Valve prosthesis 1400 of FIG. 14 is similar to valve prosthesis 300 of FIG. 3 and only the significant differences are discussed below.

Referring now to FIGS. 5, 14 and 15 together, in accordance with this embodiment, instead of using MOA stitching 374 as illustrated in FIG. 5 to attached valve inflow cylinder 356 to inflow portion 312 of stent structure 302, collage bonding is used More particularly, inflow end outer skirt 380, sometimes called a PVL tissue skirt, and valve inflow cylinder 356 are pressed together with stent structure 302 in between. This causes inflow end outer skirt 380 and valve inflow cylinder 356 to press against one another and collagen bond through inflow portion cells 336. Thus, a collagen bond 1502 is formed between inflow end outer skirt 380 and valve inflow cylinder 356, e.g., which are formed of the same natural tissue. Accordingly, stent structure 302 is embedded between inflow end outer skirt 380 and valve inflow cylinder 356 and thus attached to prosthetic valve 304.

By using collagen bonding to mount prosthetic valve 304 to stent structure 302 instead of stitches, e.g., MOA stitches 374 as illustrated in FIG. 5, damage to valve leaflets 310 from stitches is avoided.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

Further disclosed herein is the subject-matter of the following clauses:
1. A valve prosthesis comprising:
   a prosthetic valve;
   a stent structure comprising suture slots;
   an outer skirt; and
   outer skirt sutures attaching the outer skirt to the stent structure, the outer skirt sutures being located within the suture slots.
2. The valve prosthesis of Clause 1 wherein the suture slots are on an inner surface of the stent structure.
3. The valve prosthesis of Clause 2 wherein the outer skirt sutures are recessed within the suture slots to be radially outward of the inner surface.
4. The valve prosthesis of Clause 3 wherein by recessing the outer skirt sutures within the suture slots, contact between the outer skirt sutures and the prosthetic valve is prevented.
5. The valve prosthesis of Clause 1 or according to any of the preceding claims wherein the stent structure comprises an inflow portion comprising a strut, the suture slots being formed within the strut.
6. The valve prosthesis of Clause 5 wherein the strut has a length direction, a width direction, and a depth direction, the suture slots extending a depth in the depth direction less than a thickness of the strut in the depth direction.
7. The valve prosthesis of Clause 6 wherein the depth of the suture slots is greater than a thickness of the outer skirt sutures in the depth direction.
8. The valve prosthesis of Clause 6 or Clause 7 wherein the suture slots extend across a width of the strut in the width direction.
9. A valve prosthesis comprising:
   a prosthetic valve;
   a stent structure comprising suture holes;
   an outer skirt; and
   outer skirt sutures attaching the outer skirt to the stent structure, the outer skirt sutures being located within the suture holes.
10. The valve prosthesis of Clause 9 wherein the suture holes are radially outward of an inner surface of the stent structure.
11. The valve prosthesis of Clause 10 wherein the outer skirt sutures are radially outward of the inner surface.
12. The valve prosthesis of Clause 11 wherein by locating the outer skirt sutures within the suture holes, contact between the outer skirt sutures and the prosthetic valve is prevented.
13. The valve prosthesis of Clause 9 or according to any of the Clauses 9 to 12 wherein the stent structure comprises an inflow portion comprising a strut, the suture holes being formed within the strut.
14. The valve prosthesis of Clause 13 wherein the strut has a length direction, a width direction, and a depth direction, the suture holes extending in the width direction through the strut.
15. The valve prosthesis of Clause 9 or according to any of the Clauses 9 to 14 wherein the suture holes are larger than the outer skirt sutures.
16. A valve prosthesis comprising:
   a prosthetic valve comprising valve leaflets and a valve inflow cylinder proximal of the valve leaflets;
   a stent structure comprising suture apertures extending radially through the stent structure; and
   tacking stitching tacking the prosthetic valve to the stent structure, the tacking stitching being located within the suture apertures.
17. The valve prosthesis of Clause 16 wherein the stent structure comprises a node having a suture aperture of the suture apertures therein.
18. The valve prosthesis of Clause 16 or Clause 17 wherein the stent structure comprises a strut having a suture aperture of the suture apertures therein.
19. The valve prosthesis of Clause 16 or according to any of the Clauses 16 to 18 wherein the valve leaflets are defined by cusps, the tacking stitching being provided on the valve inflow cylinder adjacent and proximal to the cusps.

## Claims

1. A valve prosthesis comprising:
a prosthetic valve;
a stent structure comprising suture slots;
an outer skirt; and
outer skirt sutures attaching the outer skirt to the stent structure, the outer skirt sutures being located within the suture slots.

2. The valve prosthesis of Claim 1 wherein the suture slots are on an inner surface of the stent structure.

3. The valve prosthesis of Claim 2 wherein the outer skirt sutures are recessed within the suture slots to be radially outward of the inner surface, in particular,
wherein by recessing the outer skirt sutures within the suture slots, contact between the outer skirt sutures and the prosthetic valve is prevented.

4. The valve prosthesis of any of the preceding claims wherein the stent structure comprises an inflow portion comprising a strut, the suture slots being formed within the strut.

5. The valve prosthesis of Claim 4 wherein the strut has a length direction, a width direction, and a depth direction, the suture slots extending a depth in the depth direction less than a thickness of the strut in the depth direction.

6. The valve prosthesis of Claim 5 wherein the depth of the suture slots is greater than a thickness of the outer skirt sutures in the depth direction, and/or
wherein the suture slots extend across a width of the strut in the width direction.

7. A valve prosthesis comprising:
a prosthetic valve;
a stent structure comprising suture holes;
an outer skirt; and
outer skirt sutures attaching the outer skirt to the stent structure, the outer skirt sutures being located within the suture holes.

8. The valve prosthesis of Claim 7 wherein the suture holes are radially outward of an inner surface of the stent structure.

9. The valve prosthesis of Claim 8 wherein the outer skirt sutures are radially outward of the inner surface, in particular,
wherein by locating the outer skirt sutures within the suture holes, contact between the outer skirt sutures and the prosthetic valve is prevented.

10. The valve prosthesis of any of the claims 7 to 9 wherein the stent structure comprises an inflow portion comprising a strut, the suture holes being formed within the strut.

11. The valve prosthesis of Claim 10 wherein the strut has a length direction, a width direction, and a depth direction, the suture holes extending in the width direction through the strut.

12. The valve prosthesis of any of the claims 7 to 11 wherein the suture holes are larger than the outer skirt sutures.

13. A valve prosthesis comprising:
a prosthetic valve comprising valve leaflets and a valve inflow cylinder proximal of the valve leaflets;
a stent structure comprising suture apertures extending radially through the stent structure; and
tacking stitching tacking the prosthetic valve to the stent structure, the tacking stitching being located within the suture apertures.

14. The valve prosthesis of Claim 13 wherein the stent structure comprises a node having a suture aperture of the suture apertures therein, and/or
wherein the stent structure comprises a strut having a suture aperture of the suture apertures therein.

15. The valve prosthesis of Claim 13 or 14 wherein the valve leaflets are defined by cusps, the tacking stitching being provided on the valve inflow cylinder adjacent and proximal to the cusps.
